# EUROPÄISCHE PATENTSCHRIFT

(11) **EP 2 608 840 B1**
(45) Veröffentlichungstag und Bekanntmachung des Hinweises auf die Patenterteilung: **25.10.2017**
(21) Anmeldenummer: 11749357.7
(22) Anmeldetag: 26.08.2011
(51) Int. Cl.: A61N 1/05, A61N 1/36

(54) **ELEKTRODE FÜR MEDIZINISCHE ANWENDUNGEN, SYSTEM MIT EINER ELEKTRODE UND VERFAHREN ZUR HERSTELLUNG EINER ELEKTRODE**
ELECTRODE FOR MEDICAL APPLICATIONS, SYSTEM HAVING AN ELECTRODE, AND METHOD FOR PRODUCING AN ELECTRODE
ÉLECTRODE POUR UTILISATIONS MÉDICALES, SYSTÈME COMPORTANT UNE ÉLECTRODE ET PROCÉDÉ DE FABRICATION D'UNE ÉLECTRODE

(30) Priorität: 26.08.2010 DE 102010035542
(43) Veröffentlichungstag der Anmeldung: 03.07.2013
(73) Patentinhaber: Acandis GmbH & Co. KG, 76327 Pfinztal (DE)
(72) Erfinder: CATTANEO, Giorgio, 76199 Karlsruhe (DE); STETT, Alfred, 72770 Reutlingen (DE); GHARABAGHI, A., 72070 Tübingen (DE)
(74) Vertreter: Kilchert, Jochen
(86) Internationale Anmeldenummer: PCT/EP2011/004308
(87) Internationale Veröffentlichungsnummer: WO 2012/025246

(56) Entgegenhaltungen:
- WO-A1-2005/110528
- WO-A2-2007/105060
- US-A1- 2007 106 359

## Beschreibung

Die Erfindung bezieht sich auf eine Elektrode für medizinische Anwendungen zur Neuromodulation und/oder Nervenstimulation und/oder neurologischen Signalerfassung, die zur Einfuhr in ein Hohlorgan eines Körpers komprimierbar und expandierbar ist und mit einer Stromversorgung gekoppelt oder koppelbar ist. Eine gattungsgemäße Elektrode ist beispielsweise aus US 5,531,779 bekannt.

Die bekannte Elektrode wird zur Behandlung von Herzfehlern eingesetzt und ist zur Übertragung relativ hoher Ströme ausgelegt, die für derartige Behandlungen erforderlich sind. Die Elektrode ist als endovaskulärer Stent ausgebildet, der aus einer Stahllegierung hergestellt und insgesamt leitfähig ist. Der gesamte Stent bildet damit die Sonde, die zur Einfuhr in den Körper komprimierbar und expandierbar ist. Zur Erzeugung eines elektrischen Feldes wird die Stentelektrode mit einer weiteren Elektrode kombiniert, die beispielsweise in der Form einer zweiten Stentelektrode ausgebildet ist, die von der ersten Stentelektrode beabstandet implantiert und unterschiedlich polarisiert wird.

Die bekannte Stentelektrode ist aus einzelnen Drähten aufgebaut, die im expandierten Zustand im wesentlichen parallel verlaufen und korbförmig ausgebildet sind. Andere auf Drähten basierende Stentstrukturen, wie beispielsweise gewebte oder geflochtene Stents werden als geeignet offenbart. Das bekannte System ermöglicht nur eine unpräzise und schlecht modulierbare Stimulation größerer Körperbereiche.

Eine ähnliche Elektrode ist aus US 2003/74039 A1 bekannt, die ebenfalls zur Herzbehandlung eingesetzt wird. Die Elektrode wird mit einer weiteren zusätzlichen Elektrode verwendet, um ein elektrisches Feld zu erzeugen.

Ferner sind Elektroden bekannt, bei denen die Elektrodenpole in ein und denselben Implantat integriert sind, sodass die zweite separate Elektrode entfallen kann. US 6,445,953 B1 offenbart beispielsweise eine Stentelektrode, an deren Außenfläche eine erste und zweite Elektrode befestigt sind, die durch ein Funksignal angesteuert werden. Ein weiteres Beispiel für eine implantierbare Elektrode, bei der die beiden Pole im selben Implantat angeordnet sind, ist aus WO 2008/094344 A1 bekannt, die einen spiralförmigen Draht offenbart, an welchem verschiedene Elektrodenbereiche bzw. Pole vorgesehen sind. Die verschiedenen Elektrodenbereiche können unabhängig voneinander angesteuert werde, sodass das mit der Elektrode erzeugbare elektrische Feld modulierbar ist. Eine ähnliche Elektrode ist aus WO2008/094789 A1 bekannt, die zur Verankerung des Elektrodendrahtes eine Stentstruktur aufweist. Diese ist mit dem Elektrodendraht verbunden, sodass durch die Expansion des Trägerstents der Elektrodendraht an die Gefäßwand gepresst wird. Der Stent dient dabei ausschließlich als mechanische Stütze. Eine weitere Elektrode aus einem Draht mit unterschiedlichen polarisierbaren Elektrodenbereichen ist aus EP 1 304 135 A2 bekannt. Ferner beschreiben die US 2007/0106359 A1 und WO 2005/110528 A1 stentartige Strukturen, die als Elektroden wirken.

Die Herstellung der vorstehend genannten Elektroden ist kompliziert, da hierfür entweder aufwendige Fügetechniken notwendig sind oder die Stromversorgung der einzelnen Elektrodenbereiche schwierig zu realisieren ist. Außerdem sind die mit der Stützstruktur verbundenen Elektroden verhältnismäßig groß, sodass einerseits die Anzahl der Elektroden limitiert ist und andererseits eine präzise Modulation der elektrischen Felder nur eingeschränkt möglich ist. Für einige der bekannten Systeme sind Leitungen erforderlich, die mit der Elektrode verbunden sind und entsprechend isoliert sein müssen, da andernfalls ein Kontakt mit der tragenden Struktur der Elektrode zu einem Kurzschluss führen würde. Die isolierten Leitungen erhöhen die Komplexität und Sperrigkeit der Systeme und erschweren die Zufuhr, insbesondere in kleine Gefäße.

Der Erfindung liegt die Aufgabe zugrunde, eine Elektrode für medizinische Anwendungen anzugeben, die eine verbesserte Modulationseigenschaft aufweist. Ferner soll mit der Erfindung ein System mit einer derartigen Elektrode und ein Verfahren zur Herstellung einer derartigen Elektrode angegeben werden.

Erfindungsgemäß wird die Aufgabe im Hinblick auf die Elektrode durch den Gegenstand des Anspruchs 1 gelöst. Hinsichtlich des Systems wird die Aufgabe durch den Gegenstand des Anspruchs 13 und hinsichtlich des Verfahrens zur Herstellung der Elektrode durch den Gegenstand des Anspruchs 15 gelöst.

Die Erfindung beruht auf dem Gedanken, eine Elektrode für medizinische Anwendungen zur Neuromodulation und/oder Nervenstimulation und/oder neurologischen Signalerfassung anzugeben, die zur Einfuhr in ein Hohlorgan eines Körpers komprimierbar und expandierbar ist und mit einer Stromzufuhr gekoppelt oder koppelbar ist. Die Elektrode umfasst eine komprimierbare und expandierbare Gitterstruktur aus Gitterstegen, die Zellen bilden, wobei die Gitterstruktur oder zumindest ein Abschnitt der Gitterstruktur mit der Stromversorgung gekoppelt oder koppelbar ist und wenigstens einen elektrisch leitenden Bereich und wenigstens einen elektrisch isolierten Bereich bildet.

Die erfindungsgemäße Elektrode unterscheidet sich von den bekannten Systemen, insbesondere von dem System gemäß US 5,531,779, dadurch, dass die Gitterstruktur aus Gitterstegen, die Zellen bilden, ausgebildet ist. Es handelt sich also nicht um eine Gitterstruktur auf der Basis von Einzeldrähten, wie bei einem Geflecht, sondern um eine Gitterstruktur auf der Basis von Gitterstegen. Die Gitterstege sind vorzugsweise in einer einzigen Wandungsebene fluchtend zueinander angeordnet. Ferner sind die Gitterstege im Unterschied zu Einzeldrähten in Kreuzungspunkten miteinander fest verbunden, insbesondere stoffschlüssig verbunden und befinden sich auch im bereich der Kreuzungspunkte in derselben Ebene. Eine Gitterstruktur aus Gitterstegen wird beispielsweise durch Ätzen oder Schneiden aus einem Vollmaterial hergestellt, wobei die Öffnungen, die die Zellen bilden, aus dem Vollmaterial herausgeschnitten werden, sodass die Gitterstege, die die Zellen begrenzen, verbleiben. Eine Alternative hierzu bildet die Herstellung der Gitterstruktur durch Abscheideverfahren, beispielsweise PVD-Verfahren, bei denen das Material der Gitterstege auf einem Substrat abgeschieden wird. Auch hierbei wird eine Gitterstruktur mit Gitterstegen erhalten, die sich von den bekannten Geflechtstrukturen in Form und Aufbau unterscheidet, da hierbei keine Drähte oder Filamente vorgesehen sind. Die Gitterstruktur kann vollständig durch das PVD-Verfahren aufgebaut werden und freitragend sein. Alternativ kann eine anderweitig aus Vollmaterial hergestellte Gitterstruktur als Trägerstruktur verwendet werden, die durch ein PVD-Verfahren beschichtet wird. Es ist möglich, stentartige Gitterstrukturen zu bilden. Diese können in an sich bekannter Weise röhrchenartig ausgebildet sein oder ausgehend von einer planaren Form gerollt sein. Ein Verfahren zur Herstellung einer planaren Gitterstruktur ist bespw. In DE 10 2006 039 840 A1 offenbart, die auf die Anmelderin zurückgeht. Die Gitterstruktur kann im implantierten Zustand planar sein oder eine andere geometrische Form aufweisen.

Ein weiterer Unterschied zu den bekannten Systemen besteht darin, dass die Gitterstruktur wenigstens einen elektrisch leitenden Bereich und wenigstens einen elektrisch isolierten Bereich bildet. Der wenigstens eine elektrisch leitende Bereich hat die Funktion wenigstens eines Elektrodenpols, bzw. die elektrisch leitenden Bereich haben die Funktion von zwei oder mehr als zwei Elektrodenpolen, die durch die Gitterstruktur selbst gebildet werden, also in die Gitterstruktur integriert sind bzw. einen Teil der Gitterstruktur bilden. Mit anderen Worten bilden Elemente der Gitterstruktur, insbesondere die Gitterstege und/oder an den einzelnen Gitterstegen jeweils angebrachte Flächenelemente, die zur Gitterstruktur gehören und mit dieser zusammen die Crimpbewegung bzw. Verformung beim Crimpen ausführen, die Pole der Elektrode. Im Unterschied dazu sind die Pole der bekannten Elektroden auf eine Außenfläche der Gitterstruktur aufgebracht, die somit nur mechanische Tragfunktion übernimmt.

Unter einem elektrisch leitenden Bereich bzw. unter einem isolierten Bereich wird ein sich in der Ebene der Gitterstruktur bzw. in der Wandungsebene erstreckender abgegrenzter Bereich der Gitterstruktur verstanden (Wandungsbereich). Bei einer stentartigen Elektrode entspricht die Wandungsebene der Mantelfläche der Elektrode. Die Abgrenzung des elektrisch leitenden Bereichs erfolgt durch wenigstens einen elektrisch isolierten Bereich, der in derselben Ebene wie der elektrisch leitende Bereich angeordnet ist und zumindest abschnittsweise den elektrisch leitenden Bereich begrenzt. Der elektrisch leitende Bereich und der elektrisch isolierte Bereich sind in derselben Ebene der Gitterstruktur angeordnet. Der elektrisch leitende Bereich und der elektrisch isolierte Bereich sind in einer Fläche der Gitterstruktur, insbesondere in einer Außenfläche und/oder in einer Innenfläche der Gitterstruktur integriert. Die Außenfläche weist im implantierten Zustand zum Gewebe hin. Bei gerollten Elektroden kann die Zuordnung des elektrisch leitenden und des elektrisch isolierten Bereichs zur Außenfläche oder zur Innenfläche einfach durch die Rollrichtung bestimmt werden. Bei einer stentartigen Gitterstruktur erstreckt sich der elektrisch leitende Bereich in Längsrichtung und/oder in Umfangsrichtung der Gitterstruktur bzw. in der Mantelfläche. Generell bilden der elektrisch leitende Bereich und der elektrisch isolierte Bereich jeweils Teilbereich der Gitterstruktur, die nebeneinander angeordnet sind. Dasselbe gilt für mehrere elektrisch leitende und isolierte Bereich.

Die erfindungsgemäße Elektrode hat verschiedene Vorteile: Da die Pole bzw. der wenigstens eine Pol der Elektrode Bestandteil der Gitterstruktur sind, sind die im Stand der Technik teilweise angewandten Fügetechniken nicht erforderlich. Durch die Ausbildung des wenigstens einen elektrisch leitenden Bereiches und wenigstens einen elektrisch isolierten Bereiches, insbesondere durch die Ausbildung mehrerer elektrisch leitender und isolierter Bereiche in Form der Gitterstruktur wird die Präzision der Nervenstimulation und der Erfassung von Signalen auf Grund von Nervenaktivitäten verbessert. Dies liegt daran, dass mit der Erfindung sehr feine elektrisch voneinander isolierte Strukturen und somit eine hohe Anzahl von unabhängig voneinander stimulierbaren Elektrodenbereichen bereit gestellt werden kann. Damit kann eine hohe Auflösung sowohl für die Stimulation bzw. Modulation von Nerven, als auch für die Signalerfassung erreicht werden. Im Rahmen der Erfindung ist es möglich, einzelne Gitterstege oder sogar Teile einzelner Gitterstege elektrisch voneinander zu isolieren, sodass eine entsprechend fein unterteilte Elektrodenstruktur gebildet wird. Durch die Verwendung der Gitterstruktur als Elektrodenbereich wird eine flexible Anpassung der Elektrodenbereiche an unterschiedliche Anwendungsfälle erreicht, da je nach Anwendungsfall größere oder kleinere Elektrodenbereiche nahezu beliebig gebildet werden können. Die Elektrode kann einen einzigen elektrisch leitenden und einen einzigen elektrisch isolierten Bereich aufweisen. Dann ist eine weitere Elektrode für den Gegenpol erforderlich. Den einzigen Pol der erfindungsgemäße Elektrode in die Gitterstruktur zu integrieren, hat den Vorteil, dass das System kompakt und robust ist, da die Gefahr der Ablösung einer Elektrode im Gegensatz zu bekannten Elektroden mit extern aufgebrachten Polen praktisch ausgeschlossen ist.

Um beide Pole in die Gitterstruktur zu integrieren, sind wenigstens zwei elektrisch leitende Bereiche vorgesehen, die unterschiedlich oder gleich polarisierbar sind.

Die Gitterstruktur weist eine Doppelfunktion auf, da diese sowohl mechanische Funktion als Trag- bzw. Stützfunktion als auch elektrische Funktion als Elektrode erfüllt. Der Platzbedarf der erfindungsgemäßen Elektrode ist daher gegenüber bekannten Elektroden verbessert, da durch die Ausbildung der Gitterstruktur selbst als Elektrode gegenüber bekannten Elektroden, bei denen die Elektrodenpole separat am Implantat angebracht sind, Raum gespart wird. Damit wird die Behandlung kleiner Gefäße, insbesondere im zerebralen Bereich durch Elektroden überhaupt ermöglicht.

Die erfindungsgemäße Elektrode ist auch für neurologische Anwendungen, insbesondere im Bereich der Sensorik, geeignet.

Bevorzugte Ausführungsformen der Erfindung sind in den Unteransprüchen angegeben. So kann zumindest ein Abschnitt der Gitterstruktur und die gesamte Gitterstruktur schichtartig aufgebaut sein. Durch den schichtartigen Aufbau kann die Gitterstruktur besonders gut in elektrisch leitende und elektrisch isolierte Bereiche unterteilt werden. Dies trägt dazu bei, dass der Platzbedarf der Elektrode verglichen mit bekannten Elektroden geringer ist. Dabei kann der elektrisch leitende und/oder der elektrisch isolierte Bereich, insbesondere der elektrisch leitende Bereich jeweils schichtartig aufgebaut sein.

Die elektrisch leitenden Bereiche und elektrisch isolierten Bereiche können entlang der Gitterstruktur an unterschiedlichen Positionen angeordnet sein, wobei die elektrisch leitenden Bereiche durch die elektrisch isolierten Bereiche voneinander getrennt sind. Alternativ oder zusätzlich kann der leitende Bereich an einer Außenfläche und/oder Innenfläche der Gitterstruktur angeordnet sein. Damit ist es möglich, die Pole der Gitterstruktur in verschiedenen Richtungen entlang der Gitterstruktur, das heißt in Richtungen entlang der Wandung der Elektrode verteilt anzuordnen bzw. zu positionieren. Der elektrisch leitende Bereich kann zusätzlich in der Außenfläche und/oder in der Innenfläche der Gitterstruktur vorgesehen und somit in Dickenrichtung der Wandung angeordnet sein. Dies bedeutet, dass die Pole der Elektrode in verschiedenen Richtungen entlang der Gitterstruktur in die Außenfläche und/oder in die Innenfläche der Gitterstruktur integriert sein können.

Die schichtartig aufgebaute Gitterstruktur weist erfindungsgemäß eine Tragschicht, wenigstens eine Isolierschicht und wenigstens eine elektrisch leitende Schicht auf. Dieser Grundaufbau der Gitterstruktur ermöglicht eine einfache Ausbildung und Trennung der elektrisch leitenden und elektrisch isolierten Bereiche. Der elektrisch leitende Bereich umfasst die elektrisch leitende Schicht, die im elektrisch isolierten Bereich weggelassen werden kann. Durch die Funktionstrennung können die mechanischen Eigenschaften der Gitterstruktur bei der Bildung der Tragschicht separat von den elektrischen Eigenschaften der Gitterstruktur bei der Bildung der Isolierschicht und der elektrisch leitenden Schicht voneinander optimiert werden. Es ist auch möglich, die gesamte Tragschicht aus einem isolierenden Material auszubilden, sodass die Tragschicht gleichzeitig die Isolierschicht bildet. Es ist ebenfalls möglich die Tragschicht aus einem elektrisch leitenden Material auszubilden, derart, dass die Tragschicht gleichzeitig die elektrisch leitende Schicht bildet und mit einer Isolierschicht versehen wird. Das bedeutet, dass wenigstens zwei Schichten, eine Isolierschicht und eine leitenden Schicht, zumindest im elektrisch leitenden Bereich ausreichen. Der elektrisch isolierte Bereich kann nur aus der Isolierschicht (einzige Schicht) bestehen. Diese Konzept ist erweiterbar, so dass der elektrisch leitende Bereich mehr als zwei Schichten und der elektrisch isolierte Bereich mehr als eine Schicht aufweist. Die weiteren Schichten können mechanische Tragschichten sowie weitere Isolierschichten und leitende Schichten umfassen.

Zur Ausbildung des elektrisch leitenden Bereiches kann die Isolierschicht zwischen der Tragschicht und der elektrisch leitenden Schicht angeordnet sein. Damit können für die Tragschicht an sich bekannte und übliche Materialien bei der Herstellung von Gitterstrukturen, beispielsweise metallische Materialien, wie Formgedächtniswerkstoffe, wie Nitinol, oder Stahl oder bioresorbierbare Materialien, wie beispielsweise Magnesium, Magnesiumlegierungen oder andere Legierungen, verwendet werden. Durch die Isolierung der elektrisch leitenden Schicht von der Tragschicht kann die elektrisch leitende Schicht mit einer Spannung beaufschlagt werden, ohne dass andere Bereiche der Gitterstruktur, wie z.B. die Tragschicht oder andere Gitterbereiche, die mit der Tragschicht des elektrisch leitenden Bereichs mechanisch verbunden sind, von Strom durchflossen werden. Im elektrisch isolierten Bereich kann die Isolierschicht die Außenschicht der Gitterstruktur bilden.

Bei einer weiteren Ausführungsform weist die Gitterstruktur wenigstens eine Leitungseinheit mit wenigstens zwei Isolierschichten auf, wobei zwischen einer ersten und einer zweiten Isolierschicht eine elektrisch leitende Schicht angeordnet ist. Durch die beidseitig isolierte Leitungseinheit können verschiedene elektrisch leitende Bereiche mit Strom versorgt werden unabhängig von der Anordnung der jeweiligen Bereiche.

Die erste Isolierschicht kann im elektrisch isolierten Bereich die Außenschicht bilden und im elektrisch leitenden Bereich mit einer weiteren elektrisch leitenden Schicht verbunden sein, die auf der ersten Isolierschicht angeordnet ist. Die elektrisch leitende Schicht bildet dabei im elektrisch leitenden Bereich die Außenschicht. Durch die alternierende Anordnung der elektrisch leitenden Schicht und der Isolierschicht kann auf einfache Weise die Abgrenzung der elektrisch isolierten und der elektrisch leitenden Bereiche erreicht werden.

Die Gitterstege können im elektrisch leitenden Bereich eine elektrisch leitende Verbreiterung aufweisen, die sich in Längsrichtung der Gitterstege erstreckt derart, dass der mit dem Hohlorgan in Berührung kommende Bereich der Gitterstege breiter ist als der vom Hohlorgan entfernte Bereich der Gitterstege. Dadurch wird die Kontaktfläche des elektrisch leitenden Bereiches zum Gewebe hin vergrößert, wodurch eine schonende Behandlung ermöglicht wird.

Bei einer weiteren bevorzugten Ausführungsform sind wenigstens ein Gittersteg, insbesondere mehrere Gitterstege jeweils mit wenigstens einer elektrisch leitenden Abdeckung verbunden, deren Längsrichtung von der Längsrichtung des Gitterstegs abweicht derart, dass sich die Abdeckung in wenigstens eine angrenzende Zelle erstreckt und diese zumindest teilweise abdeckt. Durch die Abdeckung kann die geometrische Form der jeweiligen Pole bzw. elektrisch leitenden Bereiche variiert werden. Außerdem wird auch bei dieser Ausführungsform die Kontaktfläche zum Hohlorgan hin vergrößert, wodurch eine schonende Behandlung ermöglicht wird.

Die Größe und/oder Geometrie der Abdeckungen kann zumindest teilweise gleich oder unterschiedlich sein. Die Geometrie der Abdeckung ist nahezu frei wählbar. Zusätzlich oder alternativ kann die Anzahl der Abdeckungen auf dem Umfang der Gitterstruktur und/oder in Längsrichtung der Gitterstruktur variieren. Damit können die Modulationseigenschaften der Elektrode beeinflusst werden. Die Stegverbreiterungen und die Abdeckungen können miteinander kombiniert werden. Dabei ist es möglich, dass Mischformen aus Stegverbreiterungen und Abdeckungen gebildet werden derart, dass die Stegverbreiterungen nicht durchgehend parallel zu den Stegen verlaufen, sondern sich abweichend von der Steglängsrichtung ausbauchen.

Mehrere Abdeckungen können miteinander durch die elektrisch leitende Schicht verbunden sein, insbesondere durch eine Leitungseinheit. Auf diese Weise können mehrere Abdeckungen zusammen angesteuert werden wodurch die Modulationsfähigkeit der Elektrode weiter verbessert wird.

Vorzugsweise sind verschiedene elektrisch leitende Bereiche zur Modulation elektrischer Felder miteinander verschaltbar, insbesondere variabel miteinander verschaltbar. Somit kann die Form, Anordnung und Stärke der elektrischen Felder variiert werden. Vorzugsweise sind die elektrisch leitenden Bereiche bzw. der wenigstens eine elektrisch leitende Bereich zur Erzeugung elektrischer Felder und/oder zur Signalerfassung aufgrund von Nervenaktivität angepasst. Die Elektrode kann somit zur Stimulation von Nerven und/oder zur Detektion von Nervenaktivitäten verwendet werden. Dabei werden sowohl die Stimulationsfunktion als auch die Detektionsfunktion bzw. entsprechend angepasste Elektroden sowohl für sich als auch in Kombination miteinander offenbart.

Die elektrisch leitenden Bereiche können im Prinzip nahezu beliebig in der Gitterstruktur verteilt angeordnet sein. Vorzugsweise sind mehrere elektrisch leitende Bereich in einer Richtung entlang der Gitterstruktur parallel zueinander angeordnet und jeweils durch einen elektrisch isolierten Bereich voneinander getrennt sein. Die elektrisch leitenden Bereiche können auch in einer Richtung entlang der Gitterstruktur in Serie angeordnet und jeweils durch einen elektrisch isolierten Bereich getrennt sein.

Die Ansteuerung der verschiedenen elektrisch leitenden Bereiche kann dadurch erfolgen, dass diese mit der Stromversorgung durch elektrisch leitende Schichten entlang verschiedener elektrisch voneinander isolierter Gitterstege erfolgt. Zusätzlich oder alternativ kann die Ansteuerung dadurch erfolgen, dass verschiedene elektrisch leitende Bereiche durch mehrere Leitungseinheiten in wenigstens einem Gittersteg gekoppelt oder koppelbar sind. Die Führung der Stromversorgung ist flexibel und ergibt daher größtmögliche Freiheit in der Anordnung der elektrischen Bereiche in der Gitterstruktur. Durch die Nutzung der Gitterstege als Stromleiter wird ein im Vergleich zum Stand der Technik sehr kompakter Aufbau der Elektrode erreicht, die entsprechend miniaturisiert sein kann und in entsprechend kleine Hohlräume, insbesondere in kleine Gefäße implantiert werden kann, ohne darauf eingeschränkt zu sein.

Ferner wird ein System mit einer Elektrode, wie vorstehend beschrieben, einer Stromversorgung einem Impulsgeber und einer Steuerelektronik offenbart und beansprucht, die mit der Elektrode gekoppelt oder koppelbar sind. Das bedeutet, dass sowohl die Elektrode an sich, also unabhängig vom Gesamtsystem als auch das Gesamtsystem mit einer derartigen Elektrode Gegenstand der Erfindung sind.

Das System kann wenigstens eine weitere Elektrode zur medizinischen Anwendung umfassen, die mit der erfindungsgemäßen Elektrode bzw. einer Elektrode nach einem der vorstehend genannten erfindungsgemäßen Ausführungsformen zusammen wirkt.
Dadurch werden die Anwendungsmöglichkeiten der Elektrode erweitert, wobei die Anordnung der in die Gitterstruktur integrierten elektrischen Bereiche auch im Zusammenhang mit einer separaten Elektrode vorteilhaft sind. Die weitere Elektrode kann beispielsweise eine extrakorporale Elektrode, eine weitere implantierbare Elektrode oder die Katheterspitze eines zugehörigen Zufuhrsystems sein.

Das Verfahren zur Herstellung der Elektrode beruht darauf, dass die Gitterstruktur zumindest teilweise durch physikalische Gasphasenabscheidung hergestellt wird, wobei verschiedene Materialien auf einem Substrat schichtweise abgeschieden und strukturiert werden derart, dass eine freitragende Gitterstruktur oder eine beschichtete gitterförmige Tragstruktur mit wenigstens einem elektrisch leitenden Bereich und wenigstens einem elektrisch isolierten Bereich gebildet werden. Der Einsatz von PVD-Verfahren, insbesondere von Sputterverfahren zur Herstellung der Elektrode ermöglicht die Ausbildung von Gitterstegen mit verschiedenen Schichten aus unterschiedlichen Materialien. Damit können gezielt Isolierschichten und elektrisch leitende Schichten aufgebracht und entlang der Gitterstruktur modifiziert werden, sodass nahezu beliebig angeordnete elektrische Bereiche und isolierte Bereiche ausgebildet werden können. Das erfindungsgemäße Verfahren ermöglicht also eine sehr flexible Herstellung der Elektrode, wobei die unterschiedlichen Bereiche je nach Anforderung an die Elektrode angeordnet und im Hinblick auf die geometrische Form der Bereiche ausgebildet werden können. Andere Herstellungsverfahren sind denkbar, bei denen die Isolierschichten oder elektrisch leitenden Schichten anderweitig auf die Tragschicht aufgebracht werden, beispielsweise durch Fotolithografiverfahren.

Die Erfindung wird nachfolgend anhand von Ausführungsbeispielen unter Bezug auf die beigefügten schematischen Zeichnungen näher mit weiteren Einzelheiten erläutert. In diesen zeigen:
Fig. 1 einen Längsschnitt durch die Gitterstruktur einer Elektrode nach einem erfindungsgemäßen Ausführungsbeispiel im implantierten Zustand;
Fig. 2 eine Draufsicht auf die Elektrode gemäß Figur 1
Fig. 3 zwei Ansichten eines Steges mit Abdeckung;
Fig. 4 zwei Ansichten des Gittersteges gemäß Figur 3 mit einem Querschnitt, der außerhalb der Abdeckung angeordnet ist;
Fig. 5 eine Draufsicht auf eine Elektrode nach einem weiteren erfindungsgemäßen Beispiel mit unterschiedlichen elektrischen Bereichen;
Fig. 6 ein Längsschnitt durch die Gitterstruktur einer Elektrode nach einem erfindungsgemäßen Ausführungsbeispiel im implantierten Zustand;
Fig. 7a eine Draufsicht auf eine Elektrode nach einem weiteren erfindungsgemäßen Ausführungsbeispiel;
Fig. 7b ein Ausschnitt der Gitterstruktur der Elektrode gemäß Fig. 7a;
Fig. 8 eine seitliche Ansicht der Gitterstruktur gemäß Fig. 7b mit mehreren elektrisch leitenden Schichten;
Fig. 9 einen Querschnitt durch eine Elektrode nach einem erfindungsgemäßen Ausführungsbeispiel im implantierten Zustand, wobei die Feldlinien des durch die Elektrode erzeugten elektrischen Feldes eingezeichnet sind;
Fig. 10 einen Querschnitt durch eine Elektrode nach einem weiteren erfindungsgemäßen Ausführungsbeispiel mit einer erhöhten Anzahl von Gitterstegen;
Fig. 11 die Elektrode gemäß Fig. 10 mit moduliertem elektrischem Feld;
Fig. 12 die Elektrode gemäß Fig. 10 mit einem weiteren modulierten Feld; und
Fig. 13 die Elektrode gemäß Fig. 10 mit einem weiteren modulierten elektrischen Feld;

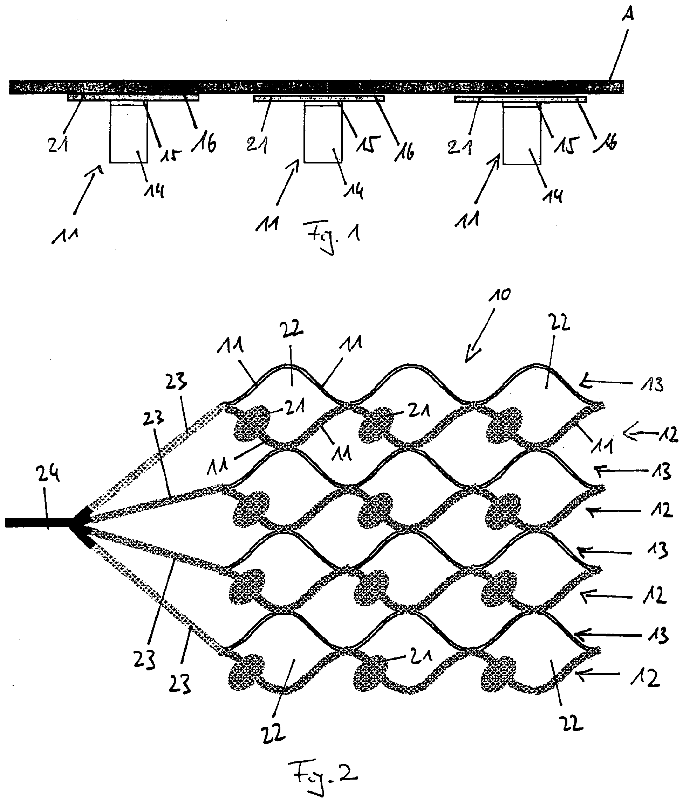

In den Figuren 1, 2 ist ein Beispiel für eine erfindungsgemäße Elektrode dargestellt, die unterschiedliche in die Gitterstruktur 10 integrierte elektrisch leitende und elektrisch isolierte Bereiche 12, 13 aufweist. Die implantierbare Elektrode für medizinische Anwendungen, insbesondere, aber nicht ausschließlich für endovaskuläre medizinische Anwendungen, kann temporär oder permanent in einem Hohlorgan eines Körpers angeordnet werden bzw. ist dazu angepasst. Die Elektrode eignet sich für den Einsatz in großen und kleinen Gefäßen, wie Herzkranzgefäßen oder intrakraniellen Gefäßen und zwar sowohl in Arterien als auch in Venen, bspw. der Carotis Arterie oder der Jugularvene. Dazu weist die Elektrode eine Gitterstruktur 10 auf, die aus Gitterstegen 11 gebildet ist. Die Gitterstege 11 bilden in an sich bekannter Weise Zellen 22. Dazu sind die Gitterstege 11 stellenweise miteinander verbunden bzw. weisen Verbinder auf, welche die Stege zur Bildung der Zellen 22 verbinden. Die Gitterstruktur 10 kann eine offen-zellige oder geschlossen-zellige Struktur aufweisen (open-cell design, closed-cell design). Derartige Strukturen sind dem Fachmann bekannt. Die auf Gitterstegen 11 basierende Gitterstruktur 10 unterscheidet sich von Drahtgeflechten, die ebenfalls Gitterstrukturen bilden, aber nicht auf der Basis von Gitterstegen, sondern auf der Basis von Filamenten oder Drähten. Gittergeflechte weisen andere mechanische Eigenschaften auf als Gitterstrukturen 10 aus Gitterstegen 11 und unterscheiden sich insbesondere in der Herstellung. Die erfindungsgemäße erreichbaren Vorteile im Hinblick auf die in die Gitterstruktur integrierten unterschiedlichen elektrisch leitenden und elektrisch isolierten Bereiche 12, 13 sind bei Geflechten nicht oder nur mit größeren Schwierigkeiten möglich. Hingegen eigenen sich die für die Herstellung von Gitterstrukturen 10 auf der Basis von Gitterstegen 11 eingesetzten Verfahren dazu, Bereiche mit unterschiedlichen elektrischen Eigenschaften in der Gitterstruktur 10 ausbilden. Dabei kommen in erster Linie Abscheidungsverfahren in Frage, mit deren Hilfe auf vergleichbar einfache Weise verschiedenartige Schichten aufgebaut werden können, die unterschiedlich elektrische Eigenschaften aufweisen. Die Erfindung ist nicht auf die Herstellung durch solche PVD-Verfahren eingeschränkt, sondern umfasst auch andere Verfahren, mit denen schichtartig aufgebaute Gitterstrukturen mit unterschiedlich elektrischen Bereichen hergestellt werden können. Denkbar sind beispielsweise Klebetechniken, mit denen verschiedene Schichten aufeinander aufgebracht werden können.

Die Form der Gitterstruktur 10 entspricht der Form eines Stents. Die Gitterstruktur 10 ist rotationssymmetrisch, insbesondere hohlzylindrisch. Bei dem Ausführungsbeispiel gemäß Figur 1, 2 handelt es sich also um eine Stentelektrode, d. h. um eine Elektrode mit einer röhrchenförmigen Gitterstruktur. Die Gitterstruktur kann auch planar hergestellt und dann gerollt, insbesondere gerollt und seitlich geschlossen (verschweißt) oder seitlich offen sein. Die Erfindung umfasst auch Gitterstrukturen mit einer anderen Form, beispielsweise Filter oder Flow Diverter. Generell kann die Gitterstruktur eine beliebige komprimierbare und expandierbare Form aufweisen, die dazu geeignet ist, durch ein Zuführsystem in ein Hohlorgan eingebracht zu werden.

Die mechanischen Eigenschaften der Elektrode einschließlich der Crimpbarkeit entsprechen den mechanischen Eigenschaften an sich bekannter Stents oder anderer bekannter endovaskulärer Implantate.

Bei der Elektrode gemäß Figur 2 handelt es sich um eine bipolare Elektrode, bei der wenigstens zwei verschiedene Elektrodenpole in die Gitterstruktur integriert sind. Die Pole sind elektrisch voneinander isoliert und entsprechen den in Figur 2 dargestellten verschiedenen elektrisch leitenden Bereiche 12. Damit ist es möglich, mit der Elektrode elektrische Felder zu erzeugen, ohne dass hierfür eine weitere Elektrode als Gegenpol erforderlich ist.

Die Pole können auch mit derselben Polarität beaufschlagt und zusammen mit einer zusätzlichen Elektrode verwendet werden, die den Gegenpol zu den auf der Elektrode gemäß Figur 2 angeordneten elektrisch leitenden Bereiche 12 bildet. Die Gegenelektrode kann extrakorporal vorgesehen sein und transkutan wirken. Es ist auch möglich, eine weitere implantierbare Elektrode gegebenenfalls auch mit mehreren elektrisch leitenden Bereichen 12 bzw. Elektrodenpolen entsprechend der Elektrode gemäß Figur 2 vorzusehen. Ferner ist es möglich, Teile eines (nicht dargestellten) Zuführsystems, beispielsweise eine Katheterspitze oder einen Führungsdraht als Gegenelektrode auszubilden.

Die Elektrode ist mit einer Stromversorgung gekoppelt oder mit einer solchen koppelbar. Dazu kann die Elektrode bzw. die Gitterstruktur 10 durch eine Leitung mit einer (nicht dargestellten) Stromversorgung verbunden sein. Die Stromversorgung der Elektrode kann als Modul zusammen mit einem Impulsgeber und einer Steuerelektronik ausgebildet sein.

In Figur 2 ist zur Verbindung der Elektrode mit der Stromversorgung bzw. dem Modul eine zentrale Versorgungsleitung 24 dargestellt, die mehrere einzelne Versorgungsleitungen 23 bzw. Stromkabel bündelt. Die Versorgungsleitungen 23 sind isoliert, und zwar sowohl innerhalb als auch außerhalb der zentralen Versorgungsleitung 24, so dass diese unabhängig voneinander mit unterschiedlichen Spannungen beaufschlagt werden können. Die zentrale Versorgungsleitung 24 kann mechanische Funktion übernehmen und beispielsweise als Betätigungsmittel für die Zufuhr oder das Wiedereinziehen der Elektrode ausgebildet sein. Die einzelnen Versorgungsleitungen 23 sind mit den unterschiedlichen elektrisch leitenden Bereichen 12 der Gitterstruktur verbunden. Im Ausführungsbeispiel gemäß Figur 2 sind vier Versorgungsleitungen 23 vorgesehen. Eine andere Anzahl entsprechend der Anzahl der anzusteuernden elektrisch leitenden Bereiche der Elektrode ist möglich.

Das Modul kann in einem ebenfalls implantierbaren Gehäuse untergebracht sein.

Andererseits ist es möglich, dass die Verbindung zwischen der Stromzufuhr und der Elektrode drahtlos erfolgt, beispielsweise durch Induktion eines elektrischen Stroms in der Gitterstruktur 10 über ein transkutanes Energieübertragungssystem (TET-System). Dazu weist die Elektrode ein Mittel zur Kopplung mit einer Stromzufuhr bzw. ein Mittel zur Stromübertragung, insbesondere zur induktiven Stromübertragung auf, das mit der Gitterstruktur, insbesondere mit dem wenigstens einen leitenden Bereich 12 verbunden ist. Dabei umfasst die Elektrode einen Empfänger, insbesondere eine Empfangsspule, die mit den elektrisch leitenden Bereichen 12 der Gitterstruktur 10 elektrisch gekoppelt ist und durch einen extrakorporalen Sender bzw. eine extrakorporale Sendespule angeregt werden kann, so dass in der Empfangsspule eine elektrische Spannung induziert wird. Zusätzlich zu der Empfangsspule kann ein Impulsgeber und eine Steuerelektronik vorgesehen sein, die mit der Gitterstruktur 10 verbunden ist. Der Impulsgeber und die Steuerelektronik können auch extrakorporal vorgesehen sein und per Funk mit der Empfangsspule bzw. einem geeigneten Empfänger kommunizieren. Außerdem ist es möglich, eine nachladbare Batterie in die Elektrode zu integrieren, die über eine Ladevorrichtung durch die Empfangsspule wieder aufgeladen werden kann. Derartige Systeme zur transkutanen Energieübertragung (TET-Systeme) sind an sich bekannt und können zusammen mit der Erfindung eingesetzt werden. Alternativ kann eine lebenslang funktionsfähige oder zumindest für die Behandlungsdauer ausgelegte Batterie verwendet werden. Es wird sowohl die Elektrode an sich mit dem Mittel zur Kopplung mit einer Stromzufuhr offenbart als auch das vorbeschriebene System, mit einer derartigen Elektrode.

Für kurzfristige Behandlungen kann die Elektrode mit Kabeln verbunden sein.

Die Stentelektrode unterscheidet sich somit von ausschließlich mechanisch wirkenden Stents u.a. dadurch, dass die Elektrode mit einer Stromversorgung gekoppelt ist oder zumindest Mittel umfasst, mit denen die Stromversorgung der elektrisch leitenden Bereiche 12 erreicht werden kann. Außerdem ist bei ausschließlich mechanisch wirkenden Stents die Gitterstruktur nicht in elektrisch leitenden und elektrisch isolierte Bereiche unterteilt, wie bei der Erfindung.

Im Rahmen der Erfindung wird sowohl die Elektrode an sich als auch das System umfassend eine Elektrode und elektrische Einrichtungen, wie beispielsweise Stromversorgung, Impulsgeber, Steuerelektronik beansprucht.

Wie in Figur 2 dargestellt, sind die unterschiedlichen Pole der Elektrode in die Gitterstruktur integriert. Dazu sind die Gitterstege 11 der Elektrode 10 so ausgebildet, dass diese bereichsweise mit einer Spannung bzw. bereichsweise mit unterschiedlichen Spannungen beaufschlagt werden können. Die stromführenden Gitterstege 11 sind von nicht stromführenden Gitterstegen 11 elektrisch isoliert. Dies wird beispielhaft an anderer Stelle anhand des schichtartigen Aufbaus der Gitterstege 11 erläutert. Die Gitterstege 11 bilden bereichsweise die Pole der Elektrode bzw. die elektrisch leitenden Bereiche 12. Eine weitere Funktion der Gitterstege 11 ist es, diskrete bzw. gesondert angeordnete elektrisch leitende Bereiche 12 mit Strom zu versorgen.

Die verschiedenen elektrisch leitenden Bereiche 12 können, wie in Figur 2 dargestellt, in verschiedenen Richtungen entlang der Gitterstruktur 10 angeordnet sein. Beispielsweise ist es möglich, die elektrisch leitenden Bereiche in Längsrichtung der stentförmigen Gitterstruktur anzuordnen. Die elektrische leitenden Bereiche 12 verlaufen dabei im Wesentlichen parallel zueinander und folgen dem Verlauf der Gitterstege 11. Daraus ergibt sich eine mehr mäandrierende Form der elektrisch leitenden Bereiche 12. Die elektrisch leitenden Bereiche 12 sind durch elektrisch isolierte Bereiche 13 voneinander getrennt, die ebenfalls parallel in Längsrichtung der stentförmigen Gitterstruktur verlaufen. Im vorliegenden Ausführungsbeispiel sind vier elektrische leitende Bereiche 12 vorgesehen. Wie in Figur 2 erkennbar, ist jedem elektrischen Bereich 12 eine eigene Versorgungsleitung 23 zugeordnet, die mit dem jeweiligen elektrischen Bereich 12 elektrisch verbunden ist. Zur Vermeidung von Kurzschlüssen und zur unabhängigen Spannungsversorgung sind die Versorgungsleitungen 23 isoliert. Die Versorgungsleitungen 23 können mehrschichtig entsprechend dem Aufbau der Gitterstruktur 10 ausgebildet sein. Die Versorgungsleitungen 23 sind mit einer Isolierung ummantelt.

Die Anzahl und Anordnung der elektrisch leitenden Bereiche ist nahezu beliebig erweiterbar. Im Extremfall weist die Elektrode einen einzigen elektrisch leitenden Bereich und einen einzigen elektrisch isolierten Bereich auf. In diesem Falle ist eine weitere Elektrode zu Erzeugung des elektrischen Feldes erforderlich. Vorzugsweise sind wenigstens zwei elektrisch leitende Bereiche in die Gitterstruktur 10 integriert, sodass die Gitterstruktur 10 die beiden Pole der Elektrode ausbildet. Die elektrisch leitenden Bereiche 12 sind durch elektrisch isolierte Bereiche 13 voneinander elektrisch getrennt.

Die elektrisch isolierten Bereiche 13 sind in Umfangsrichtung zwischen jeweils zwei elektrisch leitenden Bereiche 12 angeordnet und verlaufen somit ebenfalls parallel zueinander. Bei dem Ausführungsbeispiel gemäß Figur 2 sind also die elektrisch leitenden und elektrisch isolierten Bereiche 12, 13 entlang der Gitterstruktur an unterschiedlichen Positionen angeordnet. Dabei bedeutet die Anordnung entlang der Gitterstruktur 10, dass sich die verschiedenen Bereiche 12, 13 - im Fall einer stentförmigen Elektrode - in Längsrichtung und/oder in Umfangsrichtung der Elektrode erstrecken bzw. in diesen Richtungen angeordnet sind. Die elektrisch leitenden Bereiche 12 können auch in Dickenrichtung, d.h. in radialer Richtung der Gitterstruktur 10 angeordnet sein, sodass ein elektrisch leitender Bereich 12 entweder die Außenfläche oder eine Innenfläche der Gitterstruktur 10 bildet. Eine Kombination, wobei Innen- und Außenfläche jeweils durch elektrisch leitende Bereiche gebildet sind, die durch eine Isolierschicht voneinander getrennt sind, ist ebenfalls möglich. Eine Kombination der entlang der Gitterstruktur 10 angeordneten unterschiedlichen Bereiche 12, 13 und der in radialer Richtung angeordneten elektrisch leitenden und elektrisch isolierten Bereiche 12, 13 ist ebenfalls möglich. Bei dem Ausführungsbeispiel gemäß Figur 2 sind die elektrisch leitenden und elektrisch isolierten Bereiche 12, 13 nur entlang der Gitterstruktur angeordnet, wobei die elektrisch leitenden Bereiche 12 die Außenfläche der Gitterstruktur bilden, die im implantierten Zustand mit dem Hohlorgan der Gefäßwand in Berührung kommen. Das Lumen bzw. die Innenfläche der Gitterstruktur 10 gemäß Figur 2 ist durchgehend von der bereichsweise elektrisch aktiven Außenfläche isoliert.

Der Grundaufbau der Gitterstruktur 10 gemäß Figur 2 ist in Figur 1 dargestellt. Darin ist zu erkennen, dass die einzelnen Gitterstege 11 schichtartig aufgebaut sind. Dies gilt zumindest für die elektrisch leitenden Bereiche 12.

Die Gitterstruktur 10 der Elektrode kann mehrere Materialschichten mit unterschiedlichen Eigenschaften bzw. Funktionen aufweisen. Die Schichten können aus unterschiedlichen Materialien oder aus denselben Materialien, also denselben chemischen Zusammensetzungen bestehen und in Ihren Gefügeeigenschaften abweichen. Für alle Ausführungsbeispiele und -formen gilt, dass wenigstens eine Schicht zumindest bereichsweise die durch das PVD-Verfahren erreichbaren, in dieser Anmeldung offenbarten Gefügeeigenschaften aufweist. Es ist möglich, dass alle Schichten die gewünschten Gefügeeigenschaften aufweisen. Die Gefügeeigenschaften werden durch das PVD-Verfahren, insbesondere durch Sputtern, eingestellt. Bspw. kann der leitende Bereich, insbesondere die Außenschicht die gewünschten Gefügeeeigenschaften aufweisen und ist durch das PVD-Verfahren hergestellt. Die Tragstruktur ist auf herkömmliche Weise, bspw. durch Laserschneiden, hergestellt. Dies hat den Vorteil, dass die Außenschicht mit den verbesserten Materialeigenschaften mit dem Gewebe in Kontakt kommt. Wenn die gesamte Gitterstruktur in radialer Richtung, also alle übereinander angeordneten Schichten, durch das PVD-Verfahren hergestellt sind, werden sowohl die mechanischen als auch elektrischen Eigenschaften der Elektrode verbessert. Die Schichtdicke mit dem gesputterten Material beträgt mindestens 2%, insbesondere mindestens 5%, insbesondere mindestens 10%, insbesondere mindestens 15%, insbesondere mindestens 20%, insbesondere mindestens 25%, insbesondere mindestens 30%, insbesondere mindestens 35%, insbesondere mindestens 40%, insbesondere mindestens 45%, insbesondere mindestens 50%, insbesondere mindestens 55%, insbesondere mindestens 60%, insbesondere mindestens 65%, insbesondere mindestens 70%, insbesondere mindestens 75%, insbesondere mindestens 80%, insbesondere mindestens 85%, insbesondere mindestens 90%, insbesondere mindestens 95%, insbesondere 100% der Wandstärke der Gitterstruktur.

Die Gitterstege 11 weisen jeweils eine Tragschicht 14 auf, die bezogen auf die gekrümmte Form der Gitterstruktur 10 jeweils innen angeordnet ist und somit das Lumen der Elektrode begrenzt. Auf der Tragschicht 14 ist radial weiter außen eine Isolierschicht 15 und auf der Isolierschicht 15 eine leitende Schicht 16 aufgebracht. Die Isolierschicht 15 trennt die Tragschicht 14 von der elektrisch leitenden Schicht 16, so dass Kurzschlüsse zwischen den verschiedenen Elektrodenpolen bzw. elektrisch leitenden Bereichen 12 vermieden werden. Die Isolierschicht 15 muss nicht zwingend eine absolute elektrische Isolation in dem Sinne bilden, dass der Stromfluss zwischen der elektrisch leitenden Schicht 16 und der Tragschicht 14 vollständig unterbunden wird. Es kann für eine ordnungsgemäße Funktion der Elektrode ausreichend sein, einen Leckagestrom zuzulassen.

Für die Trennung der leitenden und isolierten Bereiche 12, 13 sind zwei Möglichkeiten denkbar: Nur die elektrisch leitende Schicht 16, nicht aber die Tragschicht 14 ist stromführend, bspw. indem nur die elektrisch leitende Schicht 16 der jeweiligen leitenden Bereiche 12 mit einer Versorgungsleitung 23 verbunden ist. Dann genügt es, in den leitenden Bereichen 12 die leitende Schicht 16 von der Tragschicht 14 zu isolieren. In den isolierten Bereichen 13 kann die Tragschicht 14 die Außenfläche bilden, also ohne Isolierschicht 15. Die Isolierschicht 15 kann natürlich auch in den isolierten Bereichen 13 vorgesehen sein. Bei der Elektrode gemäß Fig. 1,2, 3, 4 sind die Gitterstege im elektrisch isolierten Bereich 13 sind schichtartig aufgebaut, wobei die radial außen angeordnete Schicht die Isolierschicht 15 bildet. Die Tragschicht 14 kann aus einem elektrisch leitfähigem Material, bspw. aus Nitinol gebildet sein. Durch die Isolierung der Tragschicht 14 im leitenden Bereich 12, kommt es nicht zu Kurzschlüssen. Bei diesem bevorzugten Ausführungsbeispiel können die einzelnen Pole unabhängig voneinander angesteuert werden.

Alternativ kann die Tragschicht 14 vollständig, d.h. im Bereich der gesamten Gitterstruktur 10 mit Strom beaufschlagt werden, wobei die Tragschicht bereichsweise isoliert ist, also die Isolierschicht 15 trägt (isolierte Bereiche 13), und bereichsweise nicht isoliert ist. In diesen Bereichen bildet die Tragschicht 14 die Außenfläche der Gitterstruktur (leitende Bereiche 12). Dann sind alle Pole der Elektrode nur mit derselben Spannung beaufschlagbar.

Wie in Figur 1 ersichtlich, ist die Dicke der Tragschicht 14 größer als die Schichtdicke der Isolationsschicht 15 bzw. der elektrisch leitenden Schicht 16. Die Dicke der Tragschicht 14 richtet sich nach den mechanischen Eigenschaften der Elektrode bzw. der Gitterstruktur 10 der Elektrode, beispielsweise nach der Radialkraft, die die Elektrode im expandierten Zustand auf die Gefäßwand aufbringen soll. Die Dicke der Tragschicht 14 kann vom Fachmann nach Bedarf bestimmt werden. Die Dicke der übrigen Schichten 15, 16 richtet sich sowohl nach den mechanischen als auch elektrischen Anforderungen, die an die Elektrode gestellt werden.

Im einfachsten Fall bilden die Gitterstege 11 im elektrisch leitenden Bereich 12 den jeweiligen Pol der Elektrode. Dann entspricht die Kontur der elektrisch leitenden Schicht 16 der Stegkontur.

Um eine schonende Behandlung mit relativ niedrigen Stromdichten zu erreichen, ist, wie in Figur 2 dargestellt, die Kontaktfläche der Gitterstege 11 im elektrisch leitenden Bereich 12 verbreitert. Dazu weist die elektrisch leitende Schicht 16 eine Verbreiterung 20 auf, die dem Verlauf der Gitterstege 11 folgt und sich in Längsrichtung der Gitterstege 11 erstreckt derart, dass der mit dem Hohlorgan in Berührung kommende Bereich der Gitterstege 11 breiter ist als der vom Hohlorgan entfernte Bereich der Gitterstege 11, insbesondere die Tragschicht 14. Ein mögliches Verfahren zur Herstellung der Verbreiterung 20 ist in DE 10 2008 010 507 offenbart, die auf die Anmelderin zurückgeht, und die Verbindung der Gitterstege mit flexiblen Kontaktelementen durch Laserstrahl-Mikroschweißen oder Verkleben oder durch Sputtern offenbart. Im Rahmen der Erfindung wird die Verfahrensvariante, wonach die Verbreiterung 20 durch Sputtern hergestellt wird, bevorzugt. Der Inhalt der DE 10 2008 010 507 wird durch Verweis vollumfänglich in die vorliegende Anmeldung aufgenommen.

Wie in Figur 2 weiter zu erkennen, ist zusätzlich zu der Verbreiterung 20 eine Abdeckung 21 vorgesehen, die mit dem jeweiligen Gittersteg 11 verbunden ist und die Außenfläche des jeweiligen Gitterstegs 11 bildet. Dabei weicht die Längsrichtung der Abdeckung 21 von der Längsrichtung des jeweiligen Gitterstegs 11 ab derart, dass sich die Abdeckung 21 in die angrenzende Zelle 22 erstreckt und diese zumindest teilweise abdeckt. Bei dem Ausführungsbeispiel gemäß Figur 2 verläuft die Längsrichtung der Abdeckung quer zur Stegrichtung. Dabei ist jeder Zelle eine einzige Abdeckung 21 zugeordnet. Es ist auch möglich, jeder Zelle mehrere Abdeckungen 21 zuzuordnen, die entsprechend mit den stromführenden Gitterstegen verbunden sind. Es ist auch möglich, nur einen Teil der Zellen mit Abdeckungen 21 zu versehen. Im Ausführungsbeispiel gemäß Figur 2 ist die Abdeckung 21 unter einem Winkel von 90° zum jeweiligen Gittersteg, jeweils bezogen auf die Längsrichtung der Abdeckung 21 bzw. des Gitterstegs 11 angeordnet. Eine andere Ausrichtung der Abdeckung 21 ist möglich. Bei dem Ausführungsbeispiel gemäß Figur 2 ist die Abdeckung oval ausgebildet. Andere geometrische Formen, beispielsweise kreisförmige Abdeckungen sind möglich. Dasselbe gilt für die Größe der Abdeckung 21, die so angepasst werden kann, dass die Abdeckung 21 die jeweilige Zelle 22 mit verschiedenen Abdeckungsgraden überdeckt. Beispielsweise kann die Abdeckung 21 die jeweilige Zelle 22 so überdecken, dass zwischen der Abdeckung 21 und dem Gittersteg 11 des angrenzenden isolierten Bereichs 13 ein schmaler Spalt verbleibt, der ausreicht, um einen elektrische Verbindung sicher zu unterbinden.

Der Unterschied zwischen einer Abdeckung 21 und einer Verbreiterung 20 besteht darin, dass die Verbreiterung 20 dem Verlauf des Gittersteges 11 folgt, also im wesentlichen parallel zum Gittersteg 11 angeordnet ist. Die Abdeckung weist eine Längserstreckung auf, die vom Verlauf des Gittersteges 11 abweicht. Zwischenformen sind möglich, die Abdeckungen 21 und Verbreiterungen 20 kombinieren, bspw. Verbreiterungen 20, die sich seitlich bezogen auf den zugehörigen Gittersteg 11 auswölben, wobei sich die Auswölbung in eine Zelle erstreckt. Die Abdeckungen 21 und Verbreiterungen 20 bilden eine Außenschicht 17 auf der tragenden Schicht 14. Sie können aus demselben Material wie die tragende Schicht 14 gebildet sein, wobei im Fall eines leitfähigen Materials eine Isolierschicht 15 zwischen den Abdeckungen 21 und Verbreiterungen 20 einerseits und der tragenden Schicht 14 andererseits angeordnet ist.

Es ist auch möglich, die Gitterstege 11 ohne Verbreiterung 20 auszubilden. Die Breite der leitenden Schicht 16 entspricht dann der der Breite der Tragschicht 14. Wie in Figur 1 dargestellt, entspricht die Breite der Isolationsschicht 15 unterhalb der Abdeckung 21 der Breite der Tragschicht 14.

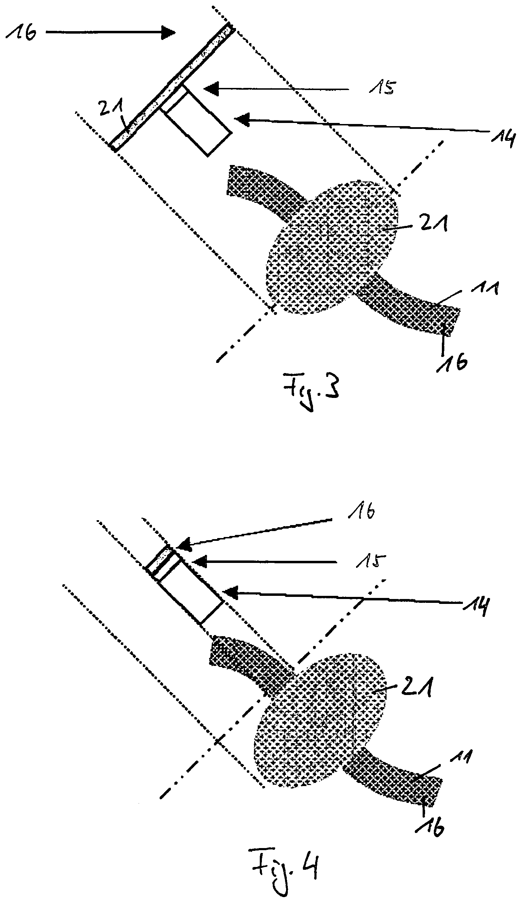

Wie in Figuren 3, 4 sowie in Fig. 2 dargestellt, verbindet die leitenden Schicht 16 (mit oder ohne Verbreiterung (20)) die Abdeckungen 21, so dass die Gitterstege 11 im elektrisch leitenden Bereich 12 die Funktion der Stromversorgung übernehmen und überdies als ein Pol der Elektrode fungieren. Es ist möglich, dass die Gitterstege nur für die Stromversorgung oder nur als Elektrodenpole ausgebildet sind. Die Figuren 3, 4 zeigen in Draufsicht einen Gittersteg 11 mit Abdeckung 21 sowie den zugehörigen Querschnitt durch den Gittersteg im Bereich der Abdeckung 21. Dasselbe gilt für Figur 4, wobei der Querschnitt durch den Gittersteg 11 neben der Abdeckung 21 dargestellt ist. Aus den Figuren 3, 4 ergibt sich, dass die Abdeckung 21 und die elektrisch leitende Schicht 16 in der gleichen Ebene des Gitterstegs 11 angeordnet sind. Die elektrisch leitende Schicht 16 ist mit der Abdeckung 21 verbunden und dient zur Stromversorgung der Abdeckung 21. Außerdem liegt die elektrische leitende Schicht 16 zum Gewebe hin frei und bildet somit selbst einen Teil der elektrisch aktiven bzw. leitenden Oberfläche des Gitterstegs 11.

Die Isolierschicht 15 erstreckt sich sowohl unter der elektrisch leitenden Schicht 16 als auch unterhalb der Abdeckung 21 und isoliert die Tragschicht 14 im gesamten elektrisch leitenden Bereich 12. In den nicht kontaktierten bzw. elektrisch isolierten Bereichen 13 ist ebenfalls die Isolierschicht 15 vorgesehen. In diesem Fall bedeckt die Isolierschicht 15 die gesamte Gitterstruktur. Alternativ kann die Isolierschicht 15 nur im elektrisch leitendem Bereich 12 vorgesehen sein, sodass im elektrisch isolierten Bereich 13 die Tragschicht 14 die Außenfläche der Gitterstruktur bildet. In diesem Fall sind die Gitterstege 11 der elektrisch leitenden Bereiche 13 anderweitig von der Stromversorgung abgekoppelt, bspw. am Verbindungsstück zu den Versorgungsleitungen 23.

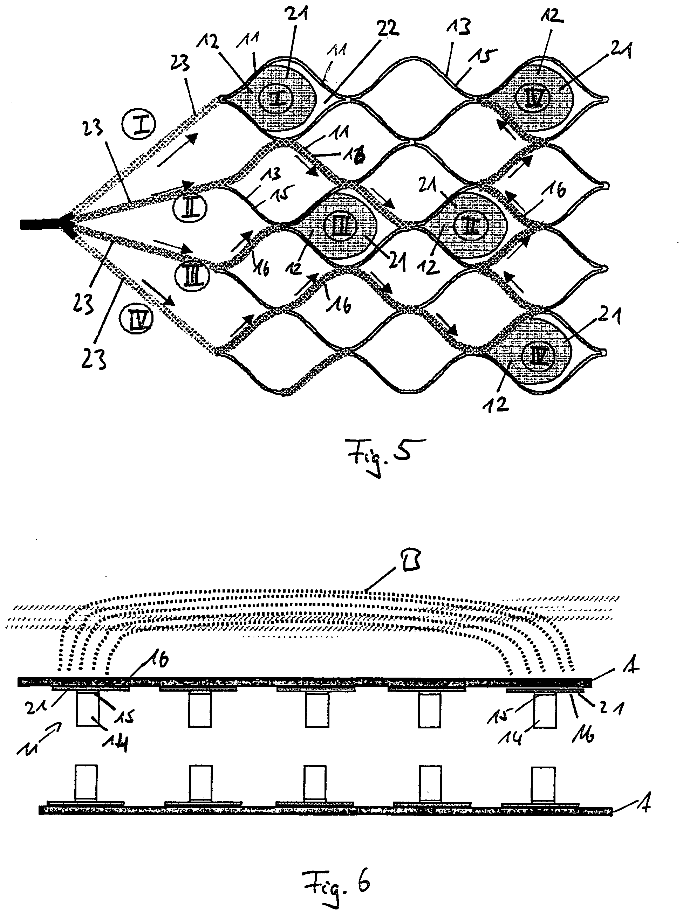

Ein Verfahren zur Herstellung der in Figur 5 dargestellten Abdeckungen 21 bzw. klappenartigen Abdeckungen 21 ist in DE 10 2009 060 228 und DE 10 2009 060 280 offenbart, die jeweils auf die Anmelderin zurückgehen. Der Inhalt dieser Anmeldungen wird durch Verweis vollumfänglich in die vorliegende Anmeldung aufgenommen.

Zusammengefasst wird bei den Ausführungsbeispielen gemäß den Figuren 1 bis 4 die elektrisch leitende Schicht 16 bzw. die Modulationsschicht stellenweise unterbrochen, so dass sich verschiedene Bereiche 12 ergeben, die voneinander isoliert sind und elektrisch leitend sind. Damit können die elektrisch leitenden Bereiche 12 unabhängig voneinander mit unterschiedlichen Spannungen oder Spannungskurven beaufschlagt werden. Die Abdeckungen 21 bzw. Stegverbreiterungen 20 erhöhen die für die Stromleitung aktive Fläche und senken dadurch die Stromdichte bzw. erhöhen bei gleicher Stromstärke die Feldstärken, was zu einer schonenden Behandlung für das umliegende Gewebe führt.

Eine weitere Möglichkeit der Anordnung der Elektrodenpole ist in Fig. 5 gezeigt:
Konkret weist die Gitterstruktur gemäß Figur 5 vier elektrisch isolierte Bereiche 12 auf, die mit I, II, III; IV bezeichnet sind. Die vier Bereiche I-IV sind mit vier verschiedenen Versorgungsleitungen 23 verbunden, die ebenfalls entsprechend jeweils mit I, II, III, IV bezeichnet sind. Der erste elektrisch leitende Bereich I ist durch eine Einzelabdeckung 21 gebildet und befindet sich am axialen Ende der Gitterstruktur 10. Die beiden elektrisch leitenden Bereiche II, III sind ebenfalls jeweils durch Einzelabdeckungen 21 gebildet und in Längsrichtung der Gitterstruktur hintereinander angeordnet. Die beiden elektrisch leitenden Bereiche II, III sind voneinander elektrisch isoliert und jeweils mit einer eigenen Versorgungsleitung II, III verbunden. In Figur 5 ist gut ersichtlich, dass die Stromversorgung des zweiten elektrischen Bereiches II seitlich am dritten elektrisch leitenden Bereich III vorbeigeführt ist und aufgrund des elektrisch isolierten Bereiches 13 von diesem getrennt ist. Der zweite und dritte elektrisch leitende Bereich II, III sind in Umfangsrichtung versetzt zum ersten elektrisch leitenden Bereich I angeordnet. Der vierte elektrisch leitende Bereich IV ist in Längsrichtung hinter dem zweiten elektrisch leitenden Bereich angeordnet und umfasst zwei Abdeckungen 21, die miteinander elektrisch verbunden sind. Dazu weisen die in Umfangsrichtung angeordneten Gitterstege 11 zwischen den beiden Abdeckungen 21 des vierten Bereichs IV jeweils eine elektrisch leitende Schicht 16 auf, die die beiden Abdeckungen 21 verbindet. Ferner sind die beiden Abdeckungen 21 des vierten Bereichs IV mit der vierten Versorgungsleitung IV durch in Längsrichtung verlaufende Gitterstege 11 der Gitterstruktur 10 verbunden, die ebenfalls eine elektrisch leitende Schicht 16 aufweisen. Die Stromversorgung des vierten Bereichs IV ist vom zweiten Bereich II, III getrennt und zwar durch elektrisch isolierte Gitterstege 11, wie in Figur 5 erkennbar. Die Stromzufuhr zu den jeweiligen Bereichen I-IV ist durch Pfeile angezeigt. Die Anordnung und die Anzahl der elektrisch leitenden Bereiche I-IV gemäß Figur 5 ist beispielhaft zu verstehen. Eine andere Anordnung und Anzahl ist möglich.

Mit dem Ausführungsbeispiel gemäß Fig. 5 wird gut verdeutlicht, wie flexibel die elektrisch leitenden Bereiche 12 in der Gitterstruktur 10 verteilt werden können.

Dabei können allgemein in Umfangsrichtung und/oder Längsrichtungen zusammenhängende elektrisch leitenden Bereiche 12 mit mehreren Abdeckungen 20 oder nur aus leitenden Gitterstegen 8 gebildete elektrisch leitenden Bereiche 12 oder zellweise begrenzte elektrisch leitenden Bereiche 12 vorgesehen sein, die in Umfangsrichtung und/oder Längsrichtung von angrenzenden elektrisch leitenden Bereichen 12 isoliert sind.

Bei dem Ausführungsbeispiel gemäß Figur 5 verlaufen die stromversorgenden Schichten 16 seitlich an den proximal, also näher an der Stromversorgung angeordneten elektrisch leitenden Bereichen 12 vorbei. Die elektrischen leitenden Schichten 16 sind dabei auf der gleichen Ebene angeordnet, wie die Abdeckungen 21. In dem mehrschichtigen System der Gitterstruktur 10 sind die elektrisch leitenden Schichten 16 und die Abdeckungen 21 so angeordnet, dass diese zur selben Schicht gehören. Die Trennung der stromführenden Gitterstege 11 und der isolierten Bereiche 13 kann beispielsweise durch Ätzen erfolgen. Dies gibt auch für alle übrigen Ausführungsbeispiele dieser Anmeldung. Es ist auch möglich, die leitenden Bereiche 12 ohne Abdeckungen 21 auszubilden, so dass die Stege 11 bzw. Elemente, die den Stegen 11 folgen, die Pole bilden.

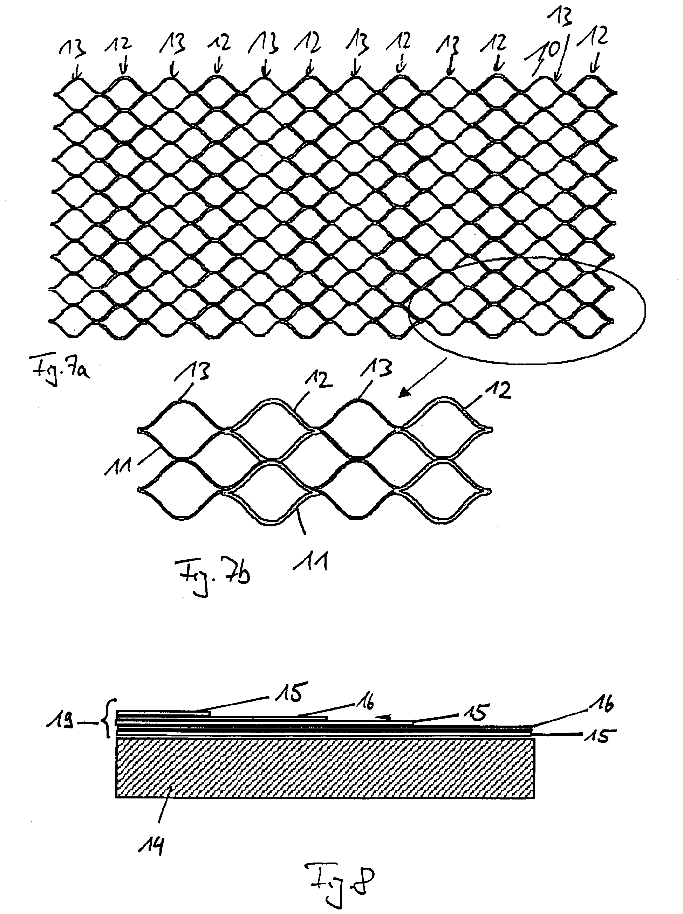

Eine weitere Möglichkeit elektrisch leitende Bereiche 12 anzusteuern bzw. mit Strom zu versorgen ist anhand des Ausführungsbeispiels gemäß den Figuren 6, 7a, 7b und Figur 8 dargestellt. Die Struktur der elektrisch leitenden Bereiche 12 besteht aus Umfangsreihen, wie in Figur 6 dargestellt, die separat voneinander angesteuert werden können. Dies ist durch die in Figur 6 dargestellten Feldlinien des elektrischen Feldes verdeutlicht, die zwischen den beiden äußeren Reihen der elektrischen Bereiche 12 verlaufen. Wie auch in Figur 7a zu erkennen, sind die elektrisch leitenden Bereiche 12 in Umfangsrichtung angeordnet und umfassen jeweils eine Reihe aus Zellen. Die zellbildenden Gitterstege 11 weisen im leitenden Bereich 12 jeweils eine leitende Schicht 16 als Außenfläche auf. Die leitenden Bereiche 12 bilden geschlossene Umfangssegmente. Offene Umfangssegmente, also Zellreihen, die sich nicht über den gesamten Umfang erstrecken sind auch möglich. Die Bereiche 12 sind in Längsrichtung voneinander beabstandet und verlaufen jeweils parallel zueinander. Die elektrisch leitenden und elektrisch isolierten Bereiche 12, 13 sind abwechselnd angeordnet. Die Stromversorgung der in Längsrichtung hintereinander angeordneten elektrisch leitenden Bereiche 12 erfolgt durch Leitungseinheiten 19, die mehrere in radialer Richtung der Gitterstege übereinander angeordnete elektrische leitende Schichten 16 aufweisen, die jeweils voneinander isoliert sind. Dabei verlaufen die leitenden Schichten 16 unter den Umfangssegmenten leitender Bereiche 12 zu den Umfangssegmenten nachfolgender leitender Bereiche 12.

Der Aufbau der Gitterstege 11 bei dem Ausführungsbeispiel gemäß Figur 7a, 7b ist in Figur 8 dargestellt, die eine seitlich Ansicht der Gitterstruktur gemäß Fig. 7a, 7b zeigt. Auf der Tragschicht 14 ist abwechselnd eine Isolierschicht 15 und eine elektrisch leitende Schicht 16 angeordnet, die eine Leitungseinheit 19 bilden. Die Außenschicht bildet eine Isolierschicht 15, die jeweils vor einem leitenden Bereich 12 endet und in diesem Bereich 12 die leitende Schicht 16 freigibt. Diese Anordnung wiederholt sich bei den aufeinanderfolgenden leitenden Bereichen 12. Bei dem Ausführungsbeispiel gemäß Figur 8 sind zwei elektrisch leitende Schichten 16 angeordnet, die sich jeweils zwischen zwei Isolierschichten 15 erstrecken. Die elektrisch leitenden Schichten 16 können somit unterhalb der elektrisch leitenden Bereiche 12 geführt werden und in Längsrichtung hintereinander angeordnete elektrisch leitende Bereiche 12 jeweils mit der Stromversorgung verbinden. Dies hat den Vorteil, dass sich, wie in Figur 7a dargestellt, durchgehende Reihen, d.h. in Umfangsrichtung durchgehende Reihen elektrisch leitender Zellen bzw. elektrisch leitender Gitterstege, die jeweils ein Zellsegment bilden, mit der Stromversorgung verbinden lassen, ohne die Reihen von elektrisch leitenden Bereiche 12 zu unterbrechen. Die Leitung des Signals bzw. die Kontaktierung der distal gelegenen elektrisch leitenden Bereiche 12 erfolgt entlang der Unterschicht, also von der radial außen angeordneten Oberfläche des Stents entfernt. Diese Konstruktion ist einfach und ermöglicht die Unterteilung der Elektrode in eine große Anzahl elektrisch leitender Umfangssegmente, die voneinander unabhängig sind. Die Anzahl der elektrisch leitenden Schichten zur Versorgung unterschiedlicher elektrisch leitender Bereiche 12 lässt sich beliebig erweitern. Somit wird eine Leitungseinheit 19 mit wenigstens zwei elektrisch leitenden Schichten, insbesondere mit 3, 4, 5, 6, 7, 8 oder mehr elektrisch leitenden Schichten offenbart.

Zusammengefasst bestehen verschiedene Anordnungsmöglichkeiten der Elektrodenpole:
Bei dem Ausführungsbeispiel gemäß Figur 2 sind die elektrisch leitenden Bereiche 12 in Längsrichtung der Gitterstruktur 10 angeordnet und in Umfangsrichtung voneinander elektrisch isoliert. Wie bei dem Ausführungsbeispiel gemäß Figur 5 dargestellt, können die elektrisch leitenden Bereiche 12 zusätzlich in Umfangsrichtung angeordnet sein und in Längsrichtung voneinander elektrisch isoliert sein. Die Anordnung der elektrisch leitenden Bereiche 12 nur in Umfangsrichtung und deren Isolierung in Längsrichtung ist in Fig. 7a gezeigt.

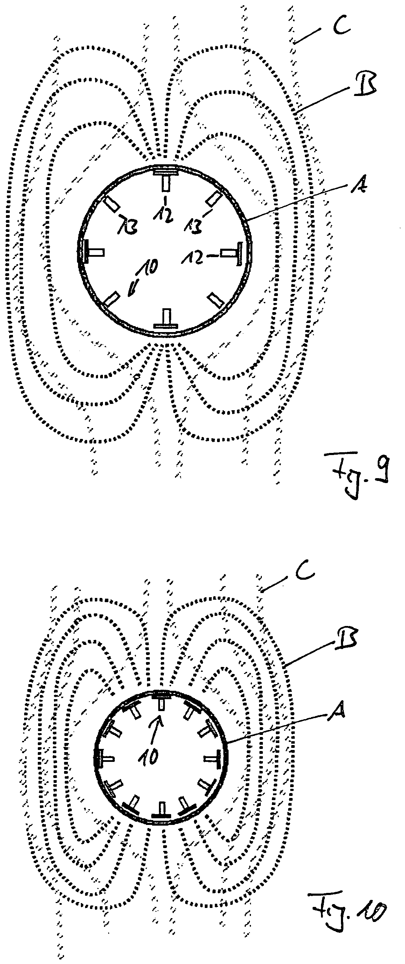

Die Funktionsweise der Elektrode gemäß den vorstehend beschriebenen Ausführungsbeispielen wird anhand der Figuren 9 bis 13, die im Prinzip dem Aufbau der Elektrode gemäß Fig. 2 entsprechen; näher erläutert, bei denen zwischen zwei gegenüberliegenden, elektrisch leitenden Bereiche 12 ein elektrisches Feld erzeugt wird. Die gute Modulationsfähigkeit der Elektrode geht beispielsweise aus Figur 9 hervor. Das resultierende elektrische Feld ist durch Feldlinien gekennzeichnet, die überwiegend parallel zu der Ebene verlaufen, die durch die beiden mit Spannung beaufschlagten elektrischen Bereiche 12 verläuft. Durch eine Erhöhung der Anzahl der Pole der Elektrode bzw. der elektrisch leitende Bereich, wie in Figur 10 dargestellt, kann die Modulation fein eingestellt werden. Außerdem ist es möglich, die Modulation an die physiologische Rückmeldung anzupassen. Durch eine Änderung der Modulationsbereiche können der Abstand und/oder die Richtung der zu stimulierenden Nerven geändert werden, ohne dass dabei die Position der Elektrode geändert werden muss.

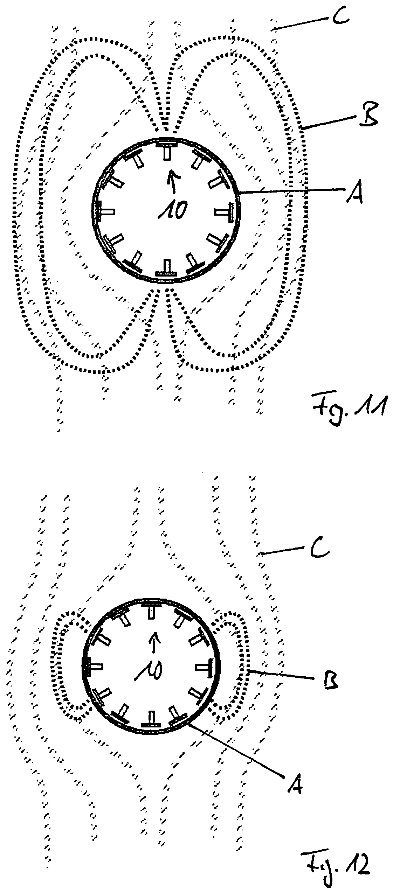

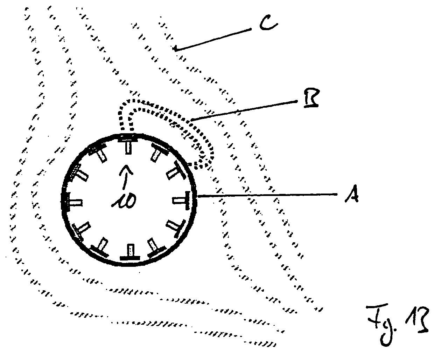

Außerdem kann die Intensität der Modulation geändert werden, ohne dabei die Spannung und damit die Stromdichte wesentlich zu variieren. Bei der Betriebsart gemäß Figur 10 wird eine große Anzahl von Nerven durch die Aktivierung mehrerer Pole bzw. elektrisch leitender Bereiche 12 erzielt. Dabei werden elektrodennahe und elektrodenferne Nerven stimuliert. Die Richtung des elektrischen Feldes ist durch die Auswahl der elektrischen Bereiche eingestellt und verläuft im Ausführungsbeispiel gemäß Figur 10 von oben nach unten. Bei der Betriebsart gemäß Figur 11 werden nur die elektrodenfernen Nerven stimuliert, die weiter distal vom Hohlorgan angeordnet sind. Die elektrodennahen, das Hohlorgan umschließenden Nerven werden nicht stimuliert. Dazu werden, wie in Figur 11 dargestellt, nur die beiden sich diametral gegenüberliegenden Pole bzw. elektrisch leitende Bereiche 12 mit Spannung beaufschlagt. Bei der Betriebsart gemäß Figur 12 werden nur die elektrodennahen, seitlich verlaufenden Nerven stimuliert. Dazu werden die elektrisch leitenden Bereiche 12 angesteuert, die in der Nähe der zu stimulierenden Nerven angeordnet sind. Wie in Figur 13 ersichtlich, kann bei einer anderen Nervenanordnung die Position der Modulationsbereiche einfach geändert werden, ohne dass dabei die Position der Elektrode geändert werden muss.

Die Modulation kann an die Rückmeldung des Organismus bzw. des Organs angepasst werden. Bei der Nervenmodulation zur Schmerzunterdrückung kann die Rückmeldung direkt durch das Empfinden des Patienten erfolgen. Dies gilt auch für Krankheiten mit offensichtlichen Anzeichen, wie Parkinson oder Epilepsie. In diesem Fall können die Parameter der Elektrode vom Arzt aktiv eingestellt werden. Bei weiteren Krankheitsbildern, wie z.B. bei Depressionen, aber auch für die vorstehend beschriebenen Krankheiten können hormonelle Untersuchungen stattfinden, die dann möglicherweise zu einer neuen Einstellung der Modulationsparameter führen. Die Stimulationserhöhung von Durchblutungen, ist beispielsweise durch die Messung der Blutströmung durch Dopplermethoden möglich. In diesen Fällen wird die Änderung der Modulationsparameter der Elektrode durch den Arzt vollzogen. Durch die Modulation können Kreislaufstörungen, wie Durchflussstörungen oder Blutdruckstörungen behandelt werden. Insbesondere kann die endovaskuläre Neuromodulation zur Behandlung von Gefäßkrankheiten wie hämorrhagischen und ischämischen Schlaganfall durch Einfluss auf Kreislaufparameter wie Blutdruck und Blutfluss, insbesondere durch Gefäßausweitung eingesetzt werden. Es ist auch möglich, das die Elektrode Bereiche aufweist, welche für die Signalerfassung angepasst sind. In diesen Bereichen werden Ströme durch die Nervenaktivität induziert und zu entsprechenden Signalen verarbeitet bzw. verstärkt. Eine Konfigurationsänderung der Modulationsbereiche kann auf diese Weise automatisch vom System durchgeführt werden. Die für die Signalerfassung erforderlichen Mittel sind an sich bekannt und können ohne weiteres mit der Elektrode kombiniert werden.

Bei den Betriebsarten gemäß den Figuren 9 bis 13 sind die Pole des elektrischen Feldes bzw. der Elektrode durch die unterschiedlichen elektrisch leitenden Bereiche 12 der Gitterstruktur 10 gebildet. Es ist auch möglich, dass ein elektrisch leitender Bereich 12, mehrere oder alle elektrisch leitenden Bereiche 12 mit der selben Spannung beaufschlagt werden und eine weitere Elektrode zur Erzeugung des elektrischen Feldes vorgesehen ist. Die weitere Elektrode kann beispielsweise extrakorporal angeordnet sein. Die zweite Elektrode kann auch ein Draht sein, der mit dem System verbunden ist oder die Spitze eines Katheters, der zum Einführen der Elektrode in den Körper verwendet wird.

Die Herstellung der Elektrode erfolgt durch ein Verfahren, bei dem die Gitterstruktur 10 zumindest teilweise durch physikalische Gasphasenabscheidung aufgebaut wird. Bei diesem Verfahren ist es möglich, verschiedene Materialien, insbesondere elektrisch isolierende Materialien und elektrisch leitende Materialien schichtweise auf einem Substrat abzuscheiden, um die im Zusammenhang mit den verschiedenen Ausführungsbeispielen beschriebenen elektrisch isolierten und elektrisch leitenden Bereiche 12, 13 zu bilden. Dabei können die durch das PVD-Verfahren hergestellten Strukturen durch an sich bekannte Ätzverfahren weiter strukturiert werden, sodass die in den Ausführungsbeispielen beschriebenen Strukturen erhalten werden. Es ist möglich, freitragende Gitterstrukturen herzustellen, die vollständig durch die schichtweise Abscheidung der Materialien aus der Gasphase gebildet sind. Alternativ kann eine herkömmlich hergestellte Stegstruktur, beispielsweise eine durch Laserschneiden hergestellte Stegstruktur durch ein PVD-Verfahren mit unterschiedlichen Materialen beschichtet werden, um die in den Ausführungsbeispielen dargestellten schichtartig aufgebauten Gitterstege zu bilden. Durch die Verwendung geeigneter Materialien können die elektrischen leitenden Bereiche 12 und die elektrisch isolierten Bereiche 13 einfach hergestellt werden. Mögliche Verfahren zur Herstellung sind in DE 102006007231 und DE 102005018731 offenbart, deren Inhalt jeweils durch Verweis vollumfänglich in die vorliegende Anmeldung aufgenommen wird.

### Bezugszeichenliste

- 10: Gitterstruktur
- 11: Gitterstege
- 12: elektrisch leitender Bereich
- 13: elektrisch isolierter Bereich
- 14: Tragschicht
- 15: Isolierschicht
- 16: elektrisch leitende Schicht
- 17: Außenschicht
- 18: Innenschicht
- 19: Leitungseinheit
- 20: Verbreiterung
- 21: Abdeckung
- 22: Zellen
- 23: Versorgungsleitungen
- 24: Zentralversorgung

## Patentansprüche

1. Elektrode für medizinische Anwendungen zur Neuromodulation und/oder Nervenstimulation und/oder neurologischen Signalerfassung, die zur Einfuhr in ein Hohlorgan eines Körpers komprimierbar und expandierbar ist und mit einer Stromversorgung gekoppelt oder koppelbar ist, und
eine komprimierbare und expandierbare Gitterstruktur (10) aus Gitterstegen (11) aufweist, die Zellen (22) bilden, wobei die Gitterstruktur (10) mit der Stromversorgung gekoppelt oder koppelbar ist und wenigstens einen elektrisch leitenden Bereich (12) und wenigstens einen elektrisch isolierten Bereich (13) bildet,
**dadurch gekennzeichnet, dass**
die Gitterstruktur (10) eine Tragschicht (14), wenigstens eine Isolierschicht (15) und wenigstens eine elektrisch leitende Schicht (16) aufweist.

2. Elektrode nach Anspruch 1,
**dadurch gekennzeichnet, dass**
zumindest ein Abschnitt der Gitterstruktur (10) oder die gesamte Gitterstruktur (10) schichtartig aufgebaut ist.

3. Elektrode nach Anspruch 1 oder 2,
**dadurch gekennzeichnet, dass**
elektrisch leitende Bereiche (12) und elektrisch isolierte Bereiche (13) entlang der Gitterstruktur (10) an unterschiedlichen Positionen angeordnet sind, wobei die elektrisch leitenden Bereiche (12) durch die elektrisch isolierten Bereiche (13) voneinander getrennt sind.

4. Elektrode nach einem der vorhergehenden Ansprüche,
**dadurch gekennzeichnet, dass**
der elektrisch leitenden Bereich (12) in einer Außenfläche und/oder in einer Innenfläche der Gitterstruktur (10) angeordnet ist.

5. Elektrode nach einem der vorhergehenden Ansprüche,
**dadurch gekennzeichnet, dass**
im elektrisch leitenden Bereich (12) die Isolierschicht zwischen der Tragschicht und der elektrisch leitenden Schicht angeordnet ist und/oder dass im elektrisch isolierten Bereich (13) die Isolierschicht die Außenschicht (17) der Gitterstruktur (10) bildet.

6. Elektrode nach einem der vorhergehenden Ansprüche,
**dadurch gekennzeichnet, dass**
die Gitterstruktur (10) wenigstens eine Leitungseinheit (19) mit wenigstens zwei Isolierschichten (15) aufweist, wobei zwischen einer ersten und einer zweiten Isolierschicht (15) eine elektrisch leitende Schicht angeordnet ist, insbesondere dass die erste Isolierschicht im elektrisch isolierten Bereich (13) die Außenschicht bildet, wobei im elektrisch leitenden Bereich (12) auf der ersten Isolierschicht eine weitere elektrisch leitende Schicht angeordnet ist, die im elektrisch leitenden Bereich (12) die Außenschicht bildet.

7. Elektrode nach einem der vorhergehenden Ansprüche,
**dadurch gekennzeichnet, dass**
die Gitterstege (11) im elektrisch leitenden Bereich (12) eine elektrisch leitende Verbreiterung (20) aufweisen, die sich in Längsrichtung der Gitterstege (11) erstreckt derart, dass der mit dem Hohlorgan in Berührung kommende Bereich der Gitterstege (11) breiter ist als der vom Hohlorgan entfernte Bereich der Gitterstege (11).

8. Elektrode nach einem der vorhergehenden Ansprüche,
**dadurch gekennzeichnet, dass**
wenigstens ein Gittersteg (11), insbesondere mehrere Gitterstege (11) jeweils mit wenigstens einer elektrisch leitenden Abdeckung (20) verbunden sind, deren Längsrichtung von der Längsrichtung des Gitterstegs abweicht derart, dass sich die Abdeckung in wenigstens eine angrenzende Zelle erstreckt und diese zumindest teilweise abdeckt.

9. Elektrode nach Anspruch 8,
**dadurch gekennzeichnet, dass**
die Größe und/oder Geometrie der Abdeckungen zumindest teilweise gleich oder unterschiedlich ist und/oder die Anzahl der Abdeckungen auf dem Umfang der Gitterstruktur (10) und/oder in Längsrichtung variiert.

10. Elektrode nach Anspruch 8 oder 9,
**dadurch gekennzeichnet, dass**
mehrere Abdeckungen miteinander durch eine elektrisch leitende Schicht oder eine Leitungseinheit verbunden sind.

11. Elektrode nach einem der vorhergehenden Ansprüche,
**dadurch gekennzeichnet, dass**
verschiedene elektrisch leitende Bereiche zur Modulation elektrischer Felder miteinander verschaltbar, insbesondere variabel miteinander verschaltbar sind und/oder dass elektrisch leitende Bereiche zur Erzeugung elektrischer Felder und/oder zur Signalerfassung auf Grund von Nervenaktivitäten angepasst sind und/oder dass mehrere elektrisch leitende Bereiche zumindest abschnittsweise entlang der Gitterstruktur (10) parallel zueinander und/oder zumindest abschnittsweise entlang der Gitterstruktur (10) in Serie angeordnet und durch elektrisch isolierte Bereiche (13) getrennt sind.

12. Elektrode nach einem der vorhergehenden Ansprüche,
**dadurch gekennzeichnet, dass**
mehrere elektrisch leitende Bereiche im Querschnitt der Gitterstruktur (10) gesehen seitlich nebeneinander angeordnet und durch elektrisch isolierte Bereiche (13) getrennt sind und/oder dass verschiedene elektrisch leitende Bereiche mit der Stromzufuhr durch elektrisch leitende Schichten entlang verschiedener elektrisch voneinander isolierter Gitterstege (11) und/oder durch mehrere Leitungseinheiten in wenigstens einem Gittersteg (11) gekoppelt oder koppelbar sind.

13. System mit einer Elektrode nach Anspruch 1, einer Stromversorgung, einem Impulsgeber und einer Steuerelektronik, die mit der Elektrode gekoppelt oder koppelbar sind.

14. System nach Anspruch 13,
**dadurch gekennzeichnet, dass**
wenigstens eine weitere Elektrode zur medizinischen Anwendung vorgesehen ist.

15. Verfahren zur Herstellung einer Elektrode nach Anspruch 1, bei dem die Gitterstruktur (10) zumindest teilweise durch physikalische Gasphasenabscheidung hergestellt wird, wobei verschiedene Materialien auf einem Substrat schichtweise abgeschieden und strukturiert werden derart, dass eine freitragende Gitterstruktur (10) oder eine beschichtete gitterförmige Tragstruktur mit wenigstens einem elektrisch leitenden Bereich (12) und wenigstens einem elektrisch isolierten Bereich (13) gebildet werden.

## Claims

1. An electrode for medical applications for neuromodulation and/or nerve stimulation and/or neurological signal detection which can be compressed and expanded for insertion into a hollow organ in a body and which is connected or can be connected to a power supply and
a compressible and expandable lattice structure (10) made up of lattice webs (11) which form cells (22), wherein the lattice structure (10) is connected or can be connected to the power supply and forms at least one electrically conductive region (12) and at least one electrically insulated region (13),
**characterized in that**
the lattice structure (10) comprises a bearing layer (14), at least one insulating layer (15) and at least one electrically conductive layer (16).

2. The electrode according to claim 1,
**characterized in that**
at least one portion of the lattice structure (10) or the entire lattice structure (10) has a layer-like construction.

3. The electrode according to claim 1 or 2,
**characterized in that**
electrically conductive regions (12) and electrically insulated regions (13) are arranged at different positions along the lattice structure (10), wherein the electrically conductive regions (12) are separated from one another by the electrically insulated regions (13).

4. The electrode according to one of the preceding claims,
**characterized in that**
the electrically conductive region (12) is arranged in an outer face and/or in an inner face of the lattice structure (10).

5. The electrode according to one of the preceding claims **characterized in that**
in the electrically conductive region (12), the insulating layer is arranged between the bearing layer and the electrically conductive layer and/or that in the electrically insulated region (13) the insulating layer forms the outer layer (17) of the lattice structure (10).

6. The electrode according to one of the preceding claims **characterized in that**
the lattice structure (10) has at least one line unit (19) with at least two insulating layers (15), wherein an electrically conductive layer is arranged between a first and a second insulating layer (15), in particular that the first insulating layer forms the outer layer in the electrically insulated region (13), wherein a further electrically conductive layer is arranged in the electrically conductive region (12) on the first insulating layer which forms the outer layer in the electrically conductive region (12).

7. The electrode according to one of the preceding claims,
**characterized in that**
the lattice webs (11) have an electrically conductive enlargement (20) in the electrically conductive region (12) which extends in the longitudinal direction of the lattice webs (11) in such a manner that the region of the lattice structures (11) coming into contact with the hollow organ is wider than the region of the lattice webs (11) remote from the hollow organ.

8. The electrode according to one of the preceding claims,
**characterized in that**
at least one lattice web (11), in particular a plurality of lattice webs (11), are each connected to at least one electrically conductive cover (20), the longitudinal direction whereof differs from the longitudinal direction of the lattice web in such a manner that the cover extends into at least one adjacent cell and covers this at least partially.

9. The electrode according to claim 8,
**characterized in that**
the size and/or geometry of the covers is at least partly equal or different and/or the number of covers on the periphery of the lattice structure (10) and/or in the longitudinal direction varies.

10. The electrode according to claim 8 or 9, **characterized in that**
a plurality of covers are connected to one another by an electrically conductive layer or a line unit.

11. The electrode according to one of the preceding claims,
**characterized in that**
different electrically conductive regions can be connected to one another for the modulation of electrical fields, in particular can be variably connected to one another and/or that electrically conductive regions are adapted for the production of electrical fields and/or for signal detection based on nerve activity and/or that a plurality of electrically conductive regions are arranged in series at least sectionally along the lattice structure (10) parallel to one another and/or at least sectionally along the lattice structure (10) and are separated by electrically insulated regions (13).

12. The electrode according to one of the preceding claims,
**characterized in that**
a plurality of electrically conductive regions are arranged laterally to one another viewed in the cross section of the lattice structure (10) and are separated by electrically insulated regions (13) and/or that different electrically conductive regions are connected or can be connected to the power supply by electrically conductive layers along different lattice webs (11) electrically insulated from one another and/or by a plurality of line units in at least one lattice web (11).

13. A system having an electrode according to claim 1, a power supply, a pulse generator and control electronics which are connected or can be connected to the electrode.

14. The system according to claim 13,
**characterized in that**
at least one further electrode is provided for the medical application.

15. A method for producing an electrode according to claim 1, wherein the lattice structure (10) is produced at least partially by physical vapor deposition, wherein different materials are deposited in layers on a substrate and structured in such a manner that a self-supporting lattice structure (10) or a coated, lattice-shaped bearing structure are formed with at least one electrically conductive region (12) and at least one electrically insulated region (13).

## Revendications

1. Electrode pour utilisations médicinales à des fins de neuromodulation et/ou stimulation nerveuse et/ou détection de signal neurologique, qui peut être comprimée et expansée en vue de l'introduction dans un organe creux et couplée ou pouvant être couplée avec une alimentation électrique, et
présente une structure de grille (10) comprimable et expansible composée de barres de grille (11), qui forment des cellules (22), dans lequel la structure de grille (10) est couplée ou peut être couplée avec l'alimentation électrique et forme au moins une zone conductrice électrique (12) et au moins une zone isolée électriquement (13),
**caractérisée en ce que**
la structure de grille (10) présente une couche porteuse (14), au moins une couche isolante (15) et au moins une couche électriquement conductrice (16).

2. Electrode selon la revendication 1, **caractérisée en ce que** au moins une portion de la structure de grille (10) ou la totalité de la structure de grille (10) a en structure en couches.

3. Electrode selon la revendication 1 ou 2, **caractérisée en ce que** les zones électriquement conductrices (12) et les zones isolées électriquement (13) sont disposées le long de la structure de grille (10) à des positions différentes, dans laquelle les zones électriquement conductrices (12) sont séparées les unes des autres par les zones isolées électriquement (13).

4. Electrode selon une des revendications précédentes, **caractérisée en ce que** la zone électriquement isolante (12) est disposée dans une surface extérieure et/ou dans une surface intérieure de la structure de grille (10).

5. Electrode selon une des revendications précédentes, **caractérisée en ce que** dans la zone électriquement conductrice (12) la couche isolante est disposée entre la couche porteuse et la couche électriquement isolante et/ou **en ce que** dans la zone électriquement isolante (13) la couche isolante forme la couche extérieure (17) de la structure de grille (10).

6. Electrode selon une des revendications précédentes, **caractérisée en ce que** la structure de grille (10) présente au moins une unité de conduction (19) avec au moins deux couches isolantes (15), dans laquelle entre une première et une seconde couche isolante (15) est disposée une couche électriquement conductrice, notamment **en ce que** la première couche isolante forme dans la zone isolée électriquement (13) la couche extérieure, dans laquelle dans la zone électriquement conductrice (12) sur la première couche isolante est disposée une couche électriquement conductrice supplémentaire, qui forme la couche extérieure dans la zone électriquement conductrice (12).

7. Electrode selon une des revendications précédentes, **caractérisée en ce que** les barres de grille (11) présentent dans la zone électriquement conductrice (12) un élargissement (20) électriquement conducteur, qui s'étend dans la direction longitudinale des barres de grille (11) de telle sorte la zone des barres de grille (11) venant en contact avec l'organe creux est plus large que la zone des barres de grille (11) éloignée de l'organe creux.

8. Electrode selon une des revendications précédentes, **caractérisée en ce que** au moins une barre de grille (11), notamment plusieurs barres de grille (11) sont respectivement reliées avec au moins un couvercle (20) électriquement conducteur, dont la direction longitudinale diffère de la direction longitudinale de la barre de grille de telle sorte que le couvercle s'étende dans au moins une cellule adjacente et la couvre au moins partiellement.

9. Electrode selon la revendication 8, **caractérisée en ce que** la taille et/ou la géométrie des couvercles est au moins partiellement similaire ou différente et/ou le nombre des couvercles sur la circonférence de la structure de grille (10) et/ou dans la direction longitudinale varie.

10. Electrode selon la revendication 8 ou 9, **caractérisée en ce que** plusieurs couvercles sont reliés les uns aux autres par une couche électriquement conductrice ou une unité de conduction.

11. Electrode selon une des revendications précédentes, **caractérisée en ce que** différentes zones de conduction électrique pour la modulation de champs électriques peuvent être connectées les unes aux autres, notamment connectées les unes aux autres de manière variable et/ou **en ce que** des zones électriquement conductrices sont adaptées pour la génération de champs électriques et/ou pour la détection de signal sur la base des activités des nerfs et/ou **en ce que** plusieurs zones électriquement conductrices sont disposées en série au moins partions le long de la structure de grille (10) parallèlement les uns aux autres et/ou au moins par portions le long de la structure de grille (10) et sont séparés par des zones isolées électriquement (13).

12. Electrode selon une des revendications précédentes, **caractérisée en ce que** plusieurs zones électriquement conductrices vues en coupe transversale de la structure de grille (10) sont disposées latéralement les unes à côté des autres et sont séparées par des zones (13) électriquement isolées et/ou **en ce que** différentes zones électriquement conductrices sont couplées ou peuvent être couplées avec l'alimentation électrique par des couches électriquement conductrices le long de différentes barres de grille (11) isolées électriquement les unes des autres et/ou par plusieurs unités de conduction dans au moins une barre de grille (11).

13. Système comportant une électrode selon la revendication 1, une alimentation électrique, un émetteur d'impulsions et une électronique de commande, qui sont couplées ou peuvent être couplées avec l'électrode.

14. Système selon la revendication 13, **caractérisé en ce que** au moins une électrode supplémentaire est prévue pour des utilisations médicinales.

15. Procédé de fabrication d'une électrode selon la revendication 1, dans lequel la structure de grille (10) est fabriquée au moins partiellement par dépôt physique en phase vapeur, dans lequel différents matériaux sont déposés en couches sur un substrat et structurées de telle sorte qu'une structure de grille (10) autoportante ou une structure en forme de grille revêtue avec au moins une couche électriquement conductrice (12) et au moins une zone isolée électriquement (13) soient formées.
